(19) **Europäisches Patentamt / European Patent Office / Office européen des brevets**

(11) **EP 2 737 895 A1**

(12) **EUROPEAN PATENT APPLICATION**

(43) Date of publication:
**04.06.2014 Bulletin 2014/23**

(21) Application number: **12382476.5**

(22) Date of filing: **30.11.2012**

(51) Int Cl.:
*A61K 9/00* *(2006.01)*     *A61K 38/18* *(2006.01)*
*A61K 9/06* *(2006.01)*     *A61K 47/42* *(2006.01)*
*A61K 9/16* *(2006.01)*     *C07K 14/485* *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Praxis Pharmaceutical, S.A.**
**01192 Arkaute (Alava) (ES)**

(72) Inventors:
• **Gainza Lafuente, Eusebio**
**01192 Arkaute (Álava) (ES)**
• **Gainza Lucea, Garazi**
**01006 Vitoria-Gasteiz (Álava) (ES)**
• **Ibarrola Moreno, Oihane**
**01192 Arkaute (Álava) (ES)**

• **Villullas Rincón, Silvia**
**01192 Arkaute (Álava) (ES)**
• **Del Pozo Pérez, Ángel**
**01192 Arkaute (Álava) (ES)**
• **Pedraz Muñoz, Jose Luis**
**01240 Alegria-Dulantxi (Álava) (ES)**
• **Hernández Martín, Rosa, María**
**01196 Etxabarri-Ibiña (Álava) (ES)**
• **Igartua Olaechea Manuela**
**01010 Vitoria-Gasteiz (Álava) (ES)**
• **Gómez Mengod, Alfredo**
**01192 Arkaute (Álava) (ES)**

(74) Representative: **Carpintero Lopez, Francisco et al**
**Herrero & Asociados, S.L.**
**Alcalá 35**
**28014 Madrid (ES)**

(54) **Microparticles with EGF, method of preparation and use**

(57) The present invention relates to microparticles comprising biocompatible polymers and epidermal growth factor. The present invention also relates to the method of preparing said microparticles and to their use in promoting wound healing.

EP 2 737 895 A1

**Description**

Field of the Invention

The present invention belongs to the field of microencapsulation. In particular, it relates to microparticles comprising biocompatible polymers and epidermal growth factor. It also relates to the method of preparing said microparticles and to their use in promoting wound healing.

Background of the Invention

Ageing population is associated with a progressive increase of chronic diseases and their complications. Wounds appear as a consequence of a combination of age-associated factors, such as venous insufficiency, occlusive arterial disease, obesity, immobility, growth factors deficiencies, diabetes mellitus, neurological defects and nutritional deficiencies, among others, which considerably deteriorate patients' quality of life, limiting their autonomy, especially when they affect the lower limbs. Morphologic skin changes in the elderly due to the natural passage of time can lead to a greater predisposition to be affected by mechanical forces caused by pressure, friction or shear, that are minor in nature or present for a brief period of time, able to generate lesions which, together with the healing problems associated with chronic diseases, prevent these lesions from curing, causing chronic, difficult-to-cure wounds. Chronic wounds represent a major health problem from an epidemiologic, economic and social point of view, and are associated with high morbidity in the elderly. Chronic wounds are a complex and heterogeneous group, although about 70% of them can be classified as pressure, diabetic or vascular ulcers, being less common those caused by inflammation, a tumor or by physical agents (by burns or radiation).

The conventional care of chronic wounds includes therapies such as hyperbaric oxygen, negative pressure, surgical debridement or dressings. These therapies are palliative and unable to guarantee adequate tissue regeneration and wound closure. The topical application of growth factors has also been used to improve healing without much success (Goldman R. Growth factors and chronic wound healing: past, present and future. Adv Skin Wound Care 2004; 17:24-35).

Epidermal growth factor (EGF) increases epidermal cell proliferation and is therefore important for the wound healing process, which depends on the mitosis and the migration of dermal fibroblasts and keratinocytes. However, proteolytic enzymes present in acute wounds considerably reduce local bioavailability of EGF and therefore the interaction time with the receptor. Therefore, in order for EGF to exert its mitogenic effect, continuous exposure of EGF with the cells for at least 6-12 hours is required to achieve effective wound healing. In this sense, a topical gel based on recombinant human epidermal growth factor (rhEGF), REGEN-D 150™, that applied twice a day until complete curing of the ulcer reduces healing time, has been authorized in India for the treatment of grade I and II diabetic foot ulcers. In the same manner, a rhEGF-based formulation, Heberprot-P, which increases granulation of high-grade diabetic foot ulcers after intralesional administration three times a week, is marketed in countries such as Argentina, Bolivia, Colombia, Cuba, Mexico and Venezuela.

Document WO 2007/087759 describes a process of formulating EGF in microspheres to prevent diabetic foot amputation, where EGF is encapsulated with a 40-60% encapsulation efficiency and the EGF release rate is 5 and 10 μg a day for 14 days. Chu et al. (Nanotechnology promotes the full-thickness diabetic wound healing effect of recombinant human epidermal growth factor in diabetic rats", Wound Repair Regen 2010; 15:499-505) describe nanoparticles with an 85.6% EGF encapsulation efficiency and a EGF content of 2%, which shows a rapid release (initial burst) during the first hour and sustained release for up to 24 hours. However, although the formulations described in Chu *et al.* promote, when compared with control groups, fibroblast proliferation and accelerate healing rate in a diabetic rat model with full thickness wounds, achieving these results requires a daily administration of the growth factor because this formulation exerts a maximum 24-hour sustained release. Repeated administrations entail a very important drawback for the patient, since increasing the number of doses leads to an increase in suffering treatment associated adverse effects, a higher probability of failing to comply with the treatment and, in cases in which the treatment is administered by means of wound local infiltration, patient rejection due to the painful feeling from injections.

Surprisingly, high EGF encapsulation efficiencies (of up to 88%) and a sustained EGF release for at least 30 days, which allows obtaining excellent healing results, even with a single administration, are achieved with the microparticles of the present invention, microparticles comprising EGF and polylactic-co-glycolic acid (PLGA) and alginate polymers. Furthermore, the sustained EGF release rate is in the order of nanograms, which also entails an advantage because prolonged exposures to higher concentrations of EGF can favor cell proliferation, which may be aggressive for the organism.

Object of the Invention

The present invention relates to a microparticle (microparticle of the invention) comprising a PLGA polymer, an alginate polymer, and epidermal growth factor.

In another aspect, the present invention relates to a pharmaceutical composition (pharmaceutical composition of the invention) comprising the microparticle of the invention and a pharmaceutically acceptable vehicle.

The present invention also relates to the microparticle of the invention for its use as a drug and to the microparticle of the invention for its use in promoting wound healing in an individual. The present invention also relates to the pharmaceutical composition of the invention for its use as a drug and to the pharmaceutical composition of the invention for its use in promoting wound healing in an individual.

In another aspect, the present invention relates to a kit comprising the microparticle of the invention or the pharmaceutical composition of the invention to promote wound healing.

Finally, in another aspect the present invention relates to a method for preparing the microparticle of the invention comprising the following steps:

a. Adding a solution of PLGA in an organic solvent (organic PLGA solution) to a solution comprising alginate and epidermal growth factor (EGF and alginate solution), and mixing the organic PLGA solution and the EGF and alginate solution,

b. Adding the emulsion obtained in step a) to an aqueous solution comprising surfactant and sodium chloride (aqueous solution), and mixing the emulsion obtained in step a) and the aqueous solution,

c. Adding the emulsion obtained in b) to a solution comprising sodium chloride and calcium chloride (chloride solution), and mixing the emulsion obtained in step b) and the chloride solution,

d. Extracting the solvent, and

e. Isolating the microparticle.

Brief Description of the Drawings

Figure 1 shows scanning electron microscopy (SEM) photographs of microparticles (A) MP4 (x2500), (B) MP5 (x1000), and (C) MP5-$\gamma$ (x1000).

Figure 2 shows a graphic representation of the percentage of sustained EGF release as a function of time in hours (A) and in days (B).

Figure 3 shows micrographs of fibroblast cultures treated with (A) serum-free medium, (B) 15 ng/mL of EGF microparticles and (C) 15 ng/ml of free EGF.

Figure 4 shows photographs of wounds created in rats treated with (A) untreated control, (B) vehicle control, (C) blank microparticle control, (D) free EGF and (E) microparticles with EGF.

Figure 5 shows micrographs of wound samples for evaluating the degree of re-epithelialization at 7, 11 and 17 days, in mice treated with (A) untreated control, (B) vehicle control, (C) blank microparticle control, (D) free EGF and (E) microparticles with EGF.

Detailed Description of the Invention

In a first aspect, the present invention relates to a microparticle (hereinafter referred to as microparticle of the invention) comprising a PLGA polymer, an alginate polymer and epidermal growth factor.

In the context of the present invention, the term microparticle refers to a free-flowing micrometric particle in which solid, liquid or gaseous materials are coated with a film of polymeric or fatty material, having a size comprised between 1 $\mu$m and 1000 $\mu$m. In a particular embodiment, the microparticle of the invention has a diameter in the range of 1 $\mu$m to 500 $\mu$m. More particularly, it has a diameter in the range of 1 $\mu$m to 100 $\mu$m, preferably the microparticle of the invention has a diameter in the range of 1 $\mu$m to 40 $\mu$m (Table 1).

PLGA is a synthetic polymer formed by associating lactic acid homopolymer (PLA) and glycolic acid homopolymer (PGA); it is highly biocompatible and toxicologically safe. In the context of the present invention, PLGA polymer is understood as any synthetic polymer formed by associating lactic acid and glycolic acid polymer, regardless of the proportion in which each of them is added in the formation. Alginates are natural polysaccharide copolymers made up of mannuronic and glucuronic acids. Although synthetic polymers are better than natural polymers in terms of purity and reproducibility, the ultrapurification of alginates gives them a low toxicity, being considered biocompatible.

In a particular embodiment, the epidermal growth factor of the microparticle of the invention is the recombinant human epidermal growth factor (rhEGF). Said rhEGF can be obtained commercially (Peprotech, Promega, Pharmchem, etc.) or produced by means of recombinant DNA technology, as described for example in Marioka-Fujimoto et al. (Modified enterotoxin signal sequences increase secretion level of the recombinant human epidermal growth factor in Eschsrichia coli. J Biol Chem. 1991 Jan 25; 266(3):1728-32) or in the patent application WO 91/18999 A1.

In a particular embodiment, the microparticle of the invention comprises between 82.7% and 99.897% (w/w) PLGA relative to the total weight of the microparticle, between 0.003% and 1.5% (w/w) EGF relative to the total weight of the microparticle and between 0.1% and 5% (w/w) alginate relative to the total weight of the microparticle. In another particular embodiment, the microparticle of the invention comprises between 0.05% and 1% (w/w) EGF relative to the total weight of the microparticle and more particularly comprises 1% (w/w) EGF relative to the total weight of the microparticle. In another particular embodiment, the microparticle of the invention comprises from 1% to 3% (w/w) alginate relative to the total weight of the microparticle, and even more particularly, it comprises 2% (w/w) alginate relative to the total weight of the microparticle.

In another particular embodiment, the microparticle of the invention optionally comprises human serum albumin (HSA) and polyethylene glycol (PEG). In a particular embodiment, the microparticle of the invention further comprises 0.1-10% (w/w) HSA relative to the total weight of the microparticle and 0.1-0.8% (v/w) PEG, preferably PEG400, relative to the total weight of the microparticle. Even more particularly, it comprises 5% (w/w) HSA and 0.5% (v/w) PEG, preferably PEG400, relative to the total weight of the microparticle.

In another particular embodiment, the microparticle of the invention is lyophilized.

In another particular embodiment, the microparticle of the invention described in the preceding paragraphs is embedded in a matrix in as a fibrin gel (fibrin matrix). The fibrin gel is based on polymerizing fibrinogen and thrombin with calcium to form a fibrin network. The microparticles of the invention embedded in a fibrin matrix have the important advantage of being able to be administered topically, for example, applying them as dressings on the contour of the wound or on the actual wound.

As previously indicated EGF increases epidermal and dermal cell proliferation and migration and is therefore important for the wound healing process. However, due to its short half life in wounds, continuous exposure of EGF with the cells is required to achieve effective healing. To solve this problem, the administered dose could not be excessively increased since this growth factor favors cell proliferation and may be aggressive for the organism, especially if it is administered systemically.

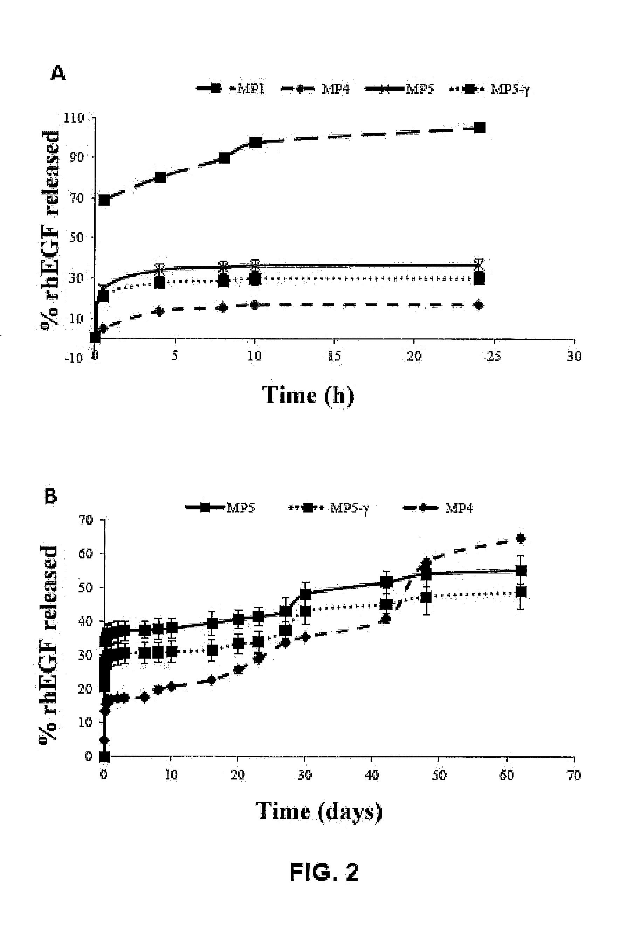

Surprisingly, the microparticle of the invention is a sustained EGF release system releasing EGF for at least 60 days (Figure 2). A release period of at least 60 days entails a sustained release over four times longer than that of the EGF microparticles described in the state of the art. Furthermore, one milligram of microparticles of the invention provides 0.2-2.5 $\mu$g of EGF/day in the rapid release phase (first 30 minutes, initial burst in which the EGF adhered to the microparticle surface is released), which allows activating the healing process, and sustained levels of 0.5-150 ng of EGF/day in the sustained release phase (slow release phase in which the EGF is released primarily by diffusion and erosion of the microparticle). The process of sustained release allows dose reduction, thereby preventing adverse effects associated both with the EGF administration dose and with those related to injecting the formulation in the lesioned area. Examples of this are inflammation, infection, systemic effects associated with EGF administration (gastric disorder, etc.).

Therefore in a particular embodiment, the microparticle of the invention defined according to any of the particular embodiments described above is characterized in that the EGF is released for at least 30 days. In another particular embodiment, the microparticle of the invention is characterized in that the EGF is released from 30 to 120 days.

In a particular embodiment, the microparticle of the invention defined according to any of the particular embodiments described above is characterized in that the EGF is released for at least 60 days. In another particular embodiment, the microparticle of the invention is characterized in that the EGF is released from 60 to 120 days.

In a particular embodiment, the microparticle of the invention defined according to any of the particular embodiments described above is characterized in that the EGF release rate is from 0.5 to 150 ng of EGF/mg of microparticles/day in the sustained release phase. In another particular embodiment, the EGF release rate is from 0.9 to 100 ng of EGF/mg of microparticles/day in the sustained release phase. In another particular embodiment, the EGF release rate is from 0.9 to 50 ng of EGF/mg of microparticles/day in the sustained release phase, in another particular embodiment it is from 5 to 80 ng of EGF/mg of microparticles/day in the sustained release phase, and in another particular embodiment it is from 5 to 50 ng of EGF/mg of microparticles/day in the sustained release phase.

Other important advantages are that the microparticle of the invention has a 60-88% encapsulation efficiency (Table 1) and that the *in vitro* biological activity of EGF is not affected by the microencapsulation process (process of forming the microparticle) or by the sterilization process by means of gamma radiations (Table 2). Therefore, the microparticle of the invention can be sterilized by gamma radiation without its biological activity and its physicochemical properties being substantially affected. With respect to other methods of sterilization, sterilization by gamma radiation offers the primary advantage of the low reactivity induced in the material to be sterilized and the ease with which the process is controlled.

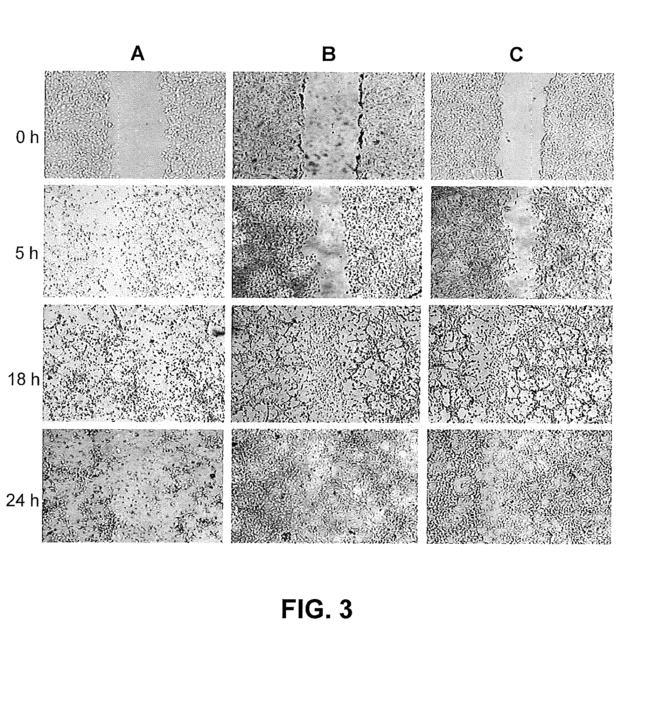

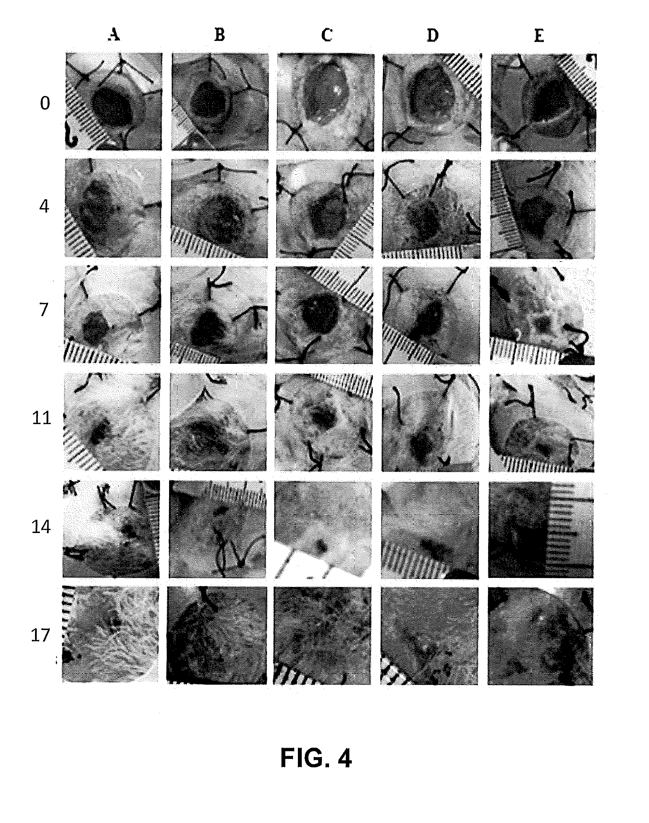

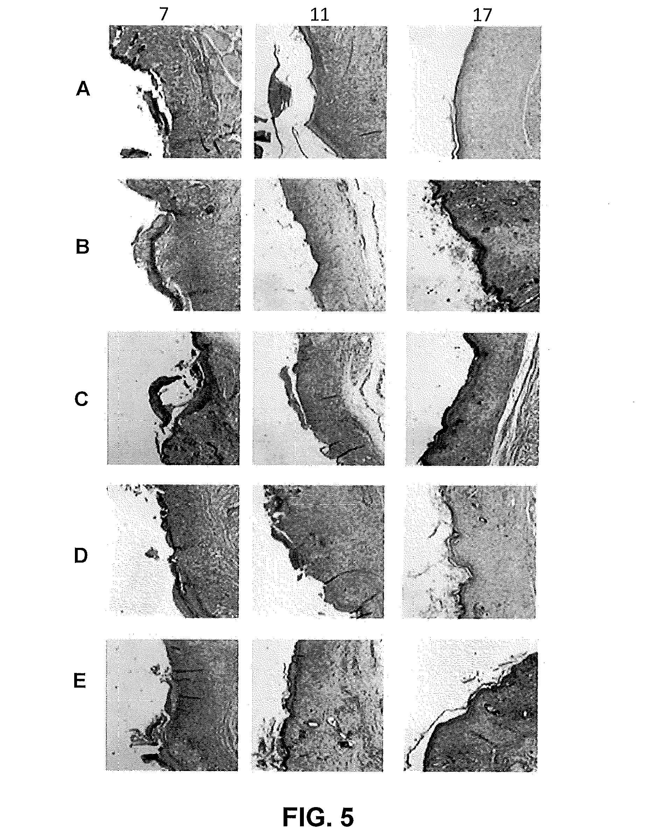

The EGF released from the microparticles of the invention is biologically active *in vitro* and is able to reduce the wound area faster than the control groups in *in vitro* healing assays (Figure 3). Even more advantageously, the EGF released from the microparticles of the invention maintains its biological activity *in vivo* and is able to promote wound healing *in vivo* by means of both intralesional applications (Figure 4, Table 3) and topical applications in preparations with fibrin (Table 4). Complete re-epithelialization of normal thickness (Figure 5) is further achieved with the microparticles of the invention, the healing being similar to physiological healing. Finally, another important advantage of the microparticles of the invention is that the microencapsulated EGF administration dose is lower than the required dose of free EGF, and a single administration (single dose) is used, which leads to fewer side effects associated with both the EGF administration and with those related to injecting the formulation in the lesioned area (Example 6).

All these features make the microparticle of the invention release bioactive EGF in a sustained manner in optimal amounts to activate epithelial cell proliferation without spreading EGF to other organs and tissues of the individual to be treated, which allows obtaining excellent healing results with single administrations in both intralesional and topical applications.

Therefore, a second aspect of the present invention relates to the use of the microparticle of the invention in preparing a drug. In a particular embodiment, it relates to the use of the microparticle of the invention in preparing a drug to promote wound healing in an individual. In a particular embodiment, the drug is applied by means of parenteral injection, and in another particular embodiment, the drug is applied by means of topical administration. In a particular embodiment, the wounds are diabetic foot ulcers, pressure ulcers and/or vascular ulcers.

The present invention also relates to the microparticle of the invention for its use as a drug. It also relates to the microparticle of the invention for its use in promoting wound healing in an individual. In a particular embodiment, it relates to the microparticle for its parenteral use to promote wound healing in an individual. And in another particular embodiment, it relates to the microparticle for its topical use to promote wound healing in an individual. In a particular embodiment, the wounds are diabetic foot ulcers, pressure ulcers and/or vascular ulcers.

In the context of the present invention, the expression to promote wound healing refers to accelerating, increasing, activating and/or initiating wound healing, leading to the wound healing in a manner similar to physiological healing, the scar tissue being mature and physiologically well organized. The term wound refers to the damage inflicted on any part of the body of an individual including but not limited to burns, cuts, trauma, etc., as well as chronic conditions such as pressure, diabetic or vascular ulcers and age-associated chronic wounds. Pressure ulcers refer to a lesion located in the skin and/or in the underlying tissue, generally on a bony prominence, as the result of pressure or pressure combined with shear. A diabetic ulcer or diabetic foot ulcer refers to a clinical disorder with an underlying neuropathic etiopathogenesis and induced by prolonged hyperglycemia in which, with or without the coexistence of ischemia, and due to a prior traumatic trigger, causes a lesion and/or ulceration of the foot which can ultimately end up with amputation. All ulcers caused by a disorder of the arterial or venous systems of the lower limbs are included under vascular ulcers. They are chronic ulcers typically found in the lower limbs and constitute a group of particularly relevant lesions from both the clinical and epidemiological viewpoints.

In a third aspect, the present invention relates to a pharmaceutical composition (pharmaceutical composition of the invention) comprising the microparticle of the invention described in the preceding paragraphs and a pharmaceutically acceptable vehicle. Pharmaceutically acceptable vehicles are widely known by the person skilled in the art. In a particular embodiment of the invention, the vehicle comprises a 0.9% w/v aqueous NaCl solution, in another particular embodiment, the vehicle further comprises 0.1-1% w/v carboxymethylcellulose, preferably 0.3% w/v carboxymethylcellulose, and in another particular embodiment, the vehicle further comprises 0.1 % v/v Tween 20. In a particular embodiment, the pharmaceutical composition of the invention further comprises one or several pharmacological agents selected from the group consisting of antiseptics, antibiotics, astringents, antifungal agents, antiviral agents, antihistamines, anti-inflammatory agents, collagen, vitamins, minerals and combinations thereof.

In a fourth aspect, the present invention relates to the use of the pharmaceutical composition of the invention in preparing a drug. In a particular embodiment, it relates to the use of the pharmaceutical composition of the invention in preparing a drug to promote wound healing in an individual. In a particular embodiment, the drug is applied by means of parenteral injection, and in another particular embodiment, the drug is applied by means of topical administration. In a particular embodiment, the wounds are diabetic foot ulcers, pressure ulcers and/or vascular ulcers.

The present invention also relates to the pharmaceutical composition of the invention for its use as a drug. It also relates to the pharmaceutical composition of the invention for its use in promoting wound healing in an individual. In a particular embodiment, it relates to the pharmaceutical composition for its parenteral use to promote wound healing in an individual. And in another particular embodiment, it relates to the pharmaceutical composition for its topical use to promote wound healing in an individual. In a particular embodiment, the wounds are diabetic foot ulcers, pressure ulcers and/or vascular ulcers.

In a fifth aspect, the present invention relates to a kit comprising the microparticle of the invention and/or the pharmaceutical composition of the invention to promote wound healing. In a particular embodiment, the kit comprises microparticles or a pharmaceutical composition comprising them and further comprises a device, preferably a syringe, for the parenteral administration of the microparticle of the invention. The parenteral administration is carried out along the perimeter of the wound.

In another particular embodiment, the kit comprises microparticles embedded in a fibrin matrix for topical administration on the contour of the wound or on the actual wound. The dosage in dressings for topical administration can be carried out in several manners known by the skilled person. Therefore in a particular embodiment of the kit of the invention, the kit comprises three solutions, a fibrinogen solution, a solution with the microparticles of the invention and a thrombin solution. The kit also comprises a sterile mold for preparing the dressing. When the dressing is needed, the fibrinogen solution and the solution with the microparticles are mixed and added to the mold, followed by the addition of the thrombin solution to these previous solutions. This mixture will be incubated at 37°C until coagulation. In another particular embodiment, the kit comprises the already prepared fibrin gel comprising the microparticles, which must be stored at 4°C and used in less than 7-10 days.

In a sixth aspect, the present invention relates to a method (hereinafter referred to as method of the invention) for preparing the microparticle of the invention described in the preceding paragraphs comprising the following steps:

a. Adding a solution of PLGA in an organic solvent (organic PLGA solution) to a solution comprising alginate and epidermal growth factor (EGF and alginate solution), and mixing the organic PLGA solution and the EGF and alginate solution,

b. Adding the emulsion obtained in step a) to an aqueous solution comprising surfactant and sodium chloride (aqueous solution), and mixing the emulsion obtained in step a) and the aqueous solution,

c. Adding the emulsion obtained in b) to a solution comprising sodium chloride and calcium chloride (chloride solution), and mixing the emulsion obtained in step b) and the chloride solution,

d. Extracting the solvent, and

e. Isolating the microparticle.

The mixing of steps a) and b) is carried out by means known by the person skilled in the art, among others, sonication, mechanical stirring, etc. In a particular embodiment, the mixing of step a) is carried out by means of applying ultrasound, and the mixing of step b) is carried out in a paddle stirrer.

The organic solvent of the organic PLGA solution is any organic solvent that dissolves PLGA and is immiscible with water, being selected in a particular embodiment from the group consisting of dichloromethane, acetonitrile, ethyl acetate, acetone, chloroform and mixtures thereof. In another particular embodiment, said solvent is dichloromethane, and in another particular embodiment, it is a mixture of dichloromethane and acetone (3:1).

The surfactant to be used is known by the person skilled in the art. In a particular embodiment, the surfactant is selected from the group consisting of, among others, potassium laurate, triethanolamine stearate, sodium lauryl sulfate, alkyl polyoxyethylene sulfates, sodium dioctyl sulfosuccinate, quaternary ammonium compounds, for example cetyltrimethylammonium bromide and lauryl dimethyl benzyl ammonium chloride, fatty alcohol polyoxyethylene ethers, sorbitan fatty acid esters, polyoxyethylene-sorbitan fatty acid esters and polyvinyl alcohol (PVA) and mixtures thereof. In a particular embodiment, the surfactant used is PVA.

In a particular embodiment of the method of the invention, the organic PLGA solution comprises 2-10% (w/v) PLGA relative to the total volume of the organic PLGA solution. In another particular embodiment, the PLGA solution comprises 3-5% (w/v) PLGA relative to the total volume of the organic PLGA solution. In another particular embodiment, the PLGA solution comprises 5% (w/v) PLGA relative to the total volume of the organic PLGA solution. In another particular embodiment, the PLGA solution comprises 3% (w/v) PLGA relative to the total volume of the organic PLGA solution.

In another particular embodiment, the EGF and alginate solution comprises 0.0015% to 0.75% (w/v) EGF relative to the total volume of the EGF and alginate solution, and 0.05% to 2.5% (w/v) alginate relative to the total volume of the EGF and alginate solution. More particularly, the EGF and alginate solution comprises 0.025% to 0.5% (w/v) EGF relative to the total volume of the EGF and alginate solution and 0.5-1.5% (w/v) alginate relative to the total volume of the EGF and alginate solution. And even more particularly, the EGF and alginate solution comprises 0.5% (w/v) EGF relative to the total volume of the EGF and alginate solution and 1% (w/v) alginate relative to the total volume of the EGF and alginate solution.

In another particular embodiment, the EGF and alginate solution further comprises 0.05-5% (w/v) HSA relative to the total volume of the EGF and alginate solution, more particularly 2.5% (w/v) HSA, and 0.15-1.16% (v/v) polyethylene glycol (PEG) relative to the total volume of the EGF and alginate solution, more particularly 0.73% (v/v) PEG400.

In another particular embodiment, the aqueous solution of step b) is a 5-10% (w/v) NaCl solution relative to the total volume of the aqueous solution, and the chloride solution of step c) comprise between 5 and 10% (w/v) sodium chloride relative to the total volume of the chloride solution and between 0.6 and 1.2 mM of calcium chloride. In another particular embodiment, the aqueous solution of step b) is a 5% (w/v) NaCl solution relative to the total volume of the aqueous solution, and the chloride solution of step c) comprise 5% sodium chloride by weight relative to the total volume of the chloride solution and 0.6 mM of calcium chloride. In another particular embodiment, the aqueous solution of step b) is a 5% (w/v) NaCl solution in a 5% (w/v) aqueous PVA solution.

Solvent extraction (step d) is carried out by methods known by the person skilled in the art, such as for example by means of vacuum extraction, evaporation or extrusion. In a particular embodiment of the method of the invention, the solvent is extracted by means of evaporation. Finally, isolating the microparticle of step e) is carried out by means of methods known by the person skilled in the art, such as centrifugation, ultracentrifugation, cross-flow filtration, vacuum filtration or vacuum evaporation.

In a particular embodiment, the microparticles isolated in step e) are optionally subjected to a lyophilization process. Said lyophilized microparticles are optionally embedded in a matrix in the form of fibrin gel (fibrin matrix). The fibrin gel is based on polymerizing fibrinogen and thrombin with calcium to form a fibrin network; therefore in a particular embodiment, the microparticles isolated in step e) are added to a fibrinogen solution to which a thrombin solution is subsequently added and immediately left to gel. The matrix in the form of fibrin gel is prepared by means known by the person skilled in the art, which briefly are based on mixing two separate solutions, one comprising fibrinogen and the other comprising thrombin. The fibrinogen and thrombin solutions can be commercially acquired separately (for example, from Sigma-Aldrich) or in a commercial kit for producing fibrin gels, such as the Tissucol ® Duo 5 mL kit (Baxter, SL, Valencia, Spain) for example.

In a final aspect, the present invention relates to the microparticle obtained by the method of the invention described above. It also relates to a composition comprising said microparticle and a pharmaceutically acceptable vehicle, as well as to the use of said microparticle or said pharmaceutical composition for preparing a drug to promote wound healing in an individual. It also relates to the microparticle obtained by the method of the invention described above and to the pharmaceutical composition comprising it together with a pharmaceutically acceptable vehicle for its use in promoting wound healing in an individual. Finally, it also relates to the kit comprising said microparticle or said pharmaceutical composition to promote wound healing.

Examples

Several specific embodiments of the invention which serve to illustrate the invention are described below.

EXAMPLE 1. Microparticle (MP) Preparation

Microparticle MP4

The microparticles MP4 were prepared as described below. For the first $w_1$/o emulsion (step a) of the method of the invention, 75 mg of PLGA were weighed and an organic 5% (w/v) PLGA solution in dichloromethane and acetone (3:1) was prepared. On the other hand, a solution of an aqueous origin was prepared with 0.1% (w/w) commercial rhEGF, 5% (w/w) HSA, 0.5% (v/w) PEG400 and 2% (w/w) alginate, relative to the amount of PLGA. The emulsion is formed when the organic solution is poured over the aqueous solution, and ultrasounds are applied for 15 seconds at an intensity of 50W (Branson® 250 sonicator). This first emulsion was then added to 15 mL of a 5% (w/v) NaCl solution in a 5% (w/v) aqueous PVA solution. This system was homogenized in a paddle stirrer at 500 rpm (Biocote® SS20 Stirrer) for 60 seconds, resulting in the second $w_1$/o/$w_2$ emulsion (emulsion resulting from step b). This mixture was incorporated in 400 mL of a 5% (w/v) NaCl solution and 0.6 mM of $CaCl_2$ in MilliQ water and maintained under stirring for 30 minutes to facilitate dichloromethane evaporation. Finally, in order to isolate the microparticles, the mixture was filtered in a vacuum pump using a 0.45 $\mu$m nylon filter. The filter was then cleaned with MilliQ water, and the microparticles that remained adhered thereto were collected. Finally, the microparticles obtained were lyophilized using a Telstar® Lyophilizer (LyoBeta) following the manufacturer's instructions.

Microparticle MP5

The protocol described for the microparticles MP4 was followed, but the rhEGF load was increased from 0.1 % to 1% to enable correctly *in vivo* dosing.

Microparticle MP5-γ (MP5 sterilized with gamma radiation)

The 1% rhEGF-loaded microparticles (MP5) were sterilized with gamma radiation using $^{60}$Co as a source of radiation. The process described in Igartua et al. (γ-Irradiation effects on biopharmaceutical properties of PLGA microspheres loaded with SPf66 synthetic vaccine. European Journal of Pharmaceutics and Biopharmaceutics 2008 6; 69(2):519-526) was followed.

EXAMPLE 2. Microparticle Characterization

Determining the microparticle size and morphology

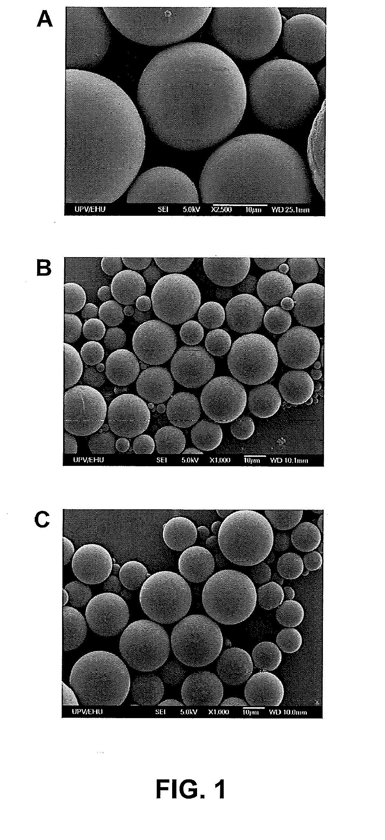

The microparticle size and morphology were determined following the protocol described in Puras et al. (Encapsulation of A$\beta$1-15 in PLGA microparticles enhances serum antibody response in mice immunized by subcutaneous and intranasal routes, European Journal of Pharmaceutical Sciences 2011; 44(3): 200-206). Briefly, the microparticle size was determined by means of laser diffraction using the Coulter® Counter LS 130 particle analyzer (Particle Size Analyzer, Amherst), according to Washington C. (Particle size analysis in pharmaceutics and other industries: theory and practice. Taylor & Fancis 1992) and morphology was analyzed by means of scanning electron microscopy (Figure 1 and Table 1).

Determining the surface charge

The surface charge is determined by means of measuring zeta potential using Zetasizer Nano series@ equipment *(Malvern Instruments user manual,* 2009), as described in Salvador et al. (Combination of immune stimulating adjuvants with poly(lactide-co-glycolide) microspheres enhances the immune response of vaccines, Vaccine, 2012, 30(3):589-596) (Table 1).

Determining encapsulation efficiency (EE%)

To determine the microencapsulation process efficiency, firstly the EGF must be extracted from the microparticles and kept in solution. To that end, 1 mg of microparticles was weighed in an Eppendorf® and 400 $\mu$L of dimethylsulfoxide (DMSO) were added. The mixture was stirred for 5 minutes at room temperature with the aid of a vortex; the DMSO thus digests the polymer and enables EGF release. Then 600 $\mu$L of ELISA diluent (composition: 0.05% Tween 20 and 0.1% BSA in DPBS) were added. Once the EGF was extracted from the microparticles, it was quantified by means of ELISA (Enzyme-linked Immunosorbent Assay). To that end, the commercial human EGF ELISA development kit by Peprotech *(*Human EGF Elisa development kit protocol, Peprotech, 2011) was used according to the manufacturer's instructions.

The encapsulation efficiency was determined by means of the following mathematical formula:

$$EE\ (\%) = (amount\ of\ EGF\ in\ the\ microparticle/amount\ of\ initial\ EGF) \times 100$$

Table 1: Results of the physicochemical characterization of the microparticles of the invention. The mean $\pm$ standard deviation is shown.

| Microparticle | Mean size ($\mu$m) | Zeta potential (mV) | Encapsulation efficiency EE (%) |
|---|---|---|---|
| MP4 | 15.47 $\pm$ 8.71 | -23.60 $\pm$ 4.6 | 88.11 $\pm$ 1.51 |
| MP5 | 12.18 $\pm$ 5.53 | -25.50 $\pm$ 9.41 | 68.82 $\pm$ 1.50 |
| MP5-$\gamma$ | 14.95 $\pm$ 6.00 | -28.50 $\pm$ 7.67 | 60.01 $\pm$ 1.10 |

As can be observed, the mean size obtained ranges between 10 and 17 $\mu$m. Variations obtained in the particle size of the different microparticles are not considered significant. Microparticles MP4, MP5 and MP5-$\gamma$ have a smooth and uniform surface without pores or irregularities. With regard to the surface charge, all the formulations have very similar negative potentials, such that relevant differences in the biopharmaceutical behavior of the particles should not to be expected. The zeta potential obtained is very negative, so the particles will tend to repel one another, preventing aggregation. Zeta potential values close to +30 mV and 30 mV are normally considered stable (Malvern Instruments user manual, 2009). Finally, with regard to the encapsulation efficiency, the EE% of microparticles MP4 is 88.11%. By increasing the EGF load from 0.1 % (MP4) to 1% (MP5), the EE% decreases to 68.82%, as was expected since increasing the EGF load is known to entail worse encapsulation. Likewise, when the microparticles are subjected to gamma radiation (MP5-$\gamma$), a slight decrease in the encapsulation efficiency (60.01 $\pm$ 1.10%) is observed, a result that can be attributed to degradation of the drug caused by the sterilization process.

EXAMPLE 3. *In vitro* Release Assay

The release profile of the EGF contained in the microparticles was determined as described in Salvador et al. (Combination of immune stimulating adjuvants with poly(lactide-co-glycolide) microspheres enhances the immune response of vaccines, Vaccine, 2012, 30(3):589-596).

The amount of EGF was quantified by means of ELISA following the method described in Example 2 for determining the EE%. The results obtained were expressed in terms of the cumulative percentage of released EGF relative to 100% of that contained in the microparticles sample as a function of time.

The results obtained are shown in Figure 2, where differences in the EGF release from microparticles MP4, MP5 and MP5-$\gamma$ are minimal. Microparticles with the components of the microparticles of the state of the art, i.e., with PLGA and EGF, were also included in this assay. Surprisingly, the EGF release from the microparticles of the invention occurs for at least 62 days, whereas microparticles with PLGA and EGF (MP1) do not have a sustained release over time, since the EGF encapsulated in them is released in less than one day (Figure 2A).

EXAMPLE 4. *In vitro* Study of the Biological Activity of EGF

The biological activity of EGF was evaluated by means of adding EGF to a cell culture in the absence of serum. Cell proliferation, increased by the presence of the factor in the medium, was quantified using the CCK-8 (Cell Counting Kit-8, Sigma-Aldrich, Saint Louise) assay as described in Zhou et al. (Effects of Leukemia Inhibitory Factor on Proliferation and Odontoblastic Differentiation of Human Dental Pulp Cells. J Endod 2011 6; 37(6):819-824.).

By knowing the concentration of EGF added to the cell medium, it is possible to determine the effective dose 50 ($ED_{50}$, minimum dose necessary for half the population to proliferate). $ED_{50}$ is directly related to the biological activity of EGF. A low $ED_{50}$ value is indicative of a higher biological activity of EGF.

The analyzed samples were the following:

- Non-encapsulated standard lyophilized EGF (lyophilized EGF).
- EGF extracted from microparticles in the release assay (MP5).
- EGF extracted from irradiated microparticles in the release assay (MP5-$\gamma$).

Table 2 shows the determined Effective Doses 50 ($ED_{50}$) from the EGF standards used (lyophilized EGF) and those from the EGF extracted from the microparticles with a 1% EGF load before and after subjecting them to the sterilization process.

Table 2.- $ED_{50}$ and Standard Deviation (SD)

|  | Concentration pg/ml | SD |
|---|---|---|
| Lyophilized EGF | 66.95 | 10.67 |
| MP5 | 54.87 | 8.58 |
| MP5-$\gamma$ | 60.78 | 11.75 |

As shown in Table 2, there are no significant differences in the biological activity of EGF when it is subjected to lyophilization. Nor are any differences observed when it is encapsulated in the microparticles of the invention, nor when the latter are sterilized by gamma radiation. Therefore, the microparticle of the invention can be sterilized by gamma radiation without substantially affecting their physicochemical properties.

EXAMPLE 5. *In vitro* Healing Assay

The *in vitro* healing assay was carried out as described in Waltera et al. (Mesenchymal stem cell-conditioned medium accelerates skin wound healing: An in vitro study of fibroblast and keratinocyte scratch assays, Experimental Cell Research 2010, 316: 1271-1281); in particular, the effect was analyzed in the following experimental groups: (A) serum-free fresh media, (B) 15 ng/mL of rhEGF obtained from the microparticles MP5 in serum-free Dulbecco's Modified Eagle's Medium (DMEM) and (C) 15 ng/ml of free rhEGF in serum-free medium (DMEM). The mean width of the gaps was calculated using ImageJ® software based on the micrographs taken at 0, 5, 18 and 24 hours from the creation of the wound (Figure 3).

As can be seen in Figure 3, the EGF released from the microparticles of the invention reduces the wound area faster than the control groups. It is further seen that the cells treated with EGF do not experience morphological changes, unlike those treated only with serum-free medium, which lose their fusiform and elongated morphology after 5 hours of treatment. It is therefore demonstrated that the EGF released from the microparticle of the invention maintains its biological activity *in vitro.*

EXAMPLE 6. *In vivo* Healing Assay

Female Wistar rats were used to perform this assay and diabetes was induced following the protocol described in Li et al. (Research of PDGF-BB Gel on the Wound Healing of Diabetic Rats and Its Pharmacodynamics. J Surg Res 2008 3; 145(1):41-8.

The induction of the wounds was carried out as described in Galiano and Michaels (Quantitative and reproducible murine model of excisional wound healing. Wound repair and regeneration 2004; 12(4):485-492).

The experimental groups analyzed were the following:

- Control group: spontaneous healing, without treatment.
- Vehicle control group: administration of 500 $\mu$L of vehicle (0.3% carboxymethylcellulose and 0.1% Tween 20 in 0.9% sodium chloride). Administration on the day of the operation.
- Blank MP control group: administration of microparticles resuspended in 500 $\mu$L of vehicle. Administration of one dose on the day of the operation.
- Free EGF group: administration of 75 $\mu$g of free EGF dissolved in 500 $\mu$L of vehicle twice a week (days 0, 4, 7, 11, 14 and 17).
- MP:EGF group: administration of MP5 (corresponding to 75 $\mu$g of EGF) resuspended in 500 $\mu$L of vehicle. Administration of one dose on the day of the operation.

In all cases, the administration was done by distributing the dose in the center and along the edges of the wound, administering the formulations subcutaneously, below the fascia of the *panniculus carnosus.*

Macroscopic analysis was performed by taking photographs of the wounds on days 0, 4, 7, 11, 14 and 17, graphically depicting the progression of the diameter of the wound as a function of time (Figure 4, Table 3).

Table 3.- Percentage of wound closure as a function of time in days. Data shown as mean $\pm$ standard deviation.

| Experimental groups | Day 4 | Day 7 | Day 11 | Day 14 | Day 17 |
|---|---|---|---|---|---|
| Control | 14.34$\pm$7.82 | 50.76$\pm$4.58 | 79.60$\pm$11.98 | 88.91$\pm$5.60 | 94.40$\pm$2.88 |
| Vehicle control | 13.06$\pm$8.88 | 47.19$\pm$6.58 | 74.24$\pm$9.96 | 89.58$\pm$4.34 | 95.43$\pm$0.62 |
| Blank MP control | 14.98$\pm$6.57 | 43.54$\pm$10.84 | 77.23$\pm$7.41 | 89.14$\pm$0.89 | 95.13$\pm$0.34 |
| Free EGF | 25.44$\pm$11.15●,▲ | 51.47$\pm$10.29 | 78.97$\pm$4.44 | 95.08$\pm$1.03 | 97.00$\pm$0.20 |
| MP:EGF | 36.43$\pm$5.45*,# | 61.06$\pm$8.69□,●,▼ | 90.29$\pm$3.60■ | 97.31$\pm$1.11 | 99.16$\pm$0.49 |

\*p<0.001 vs. control, vehicle control and blank MP control.
#p<0.01 vs. free EGF.
●p<0.01 vs. vehicle control.
▲p<0.05 vs. control and vehicle control.
□p<0.001 vs. blank MP control.
▼p<0.05 vs. control and free EGF.

As shown in Table 3, the EGF released from the microparticle of the invention maintains its biological activity *in vivo.* Four days after inducing the wounds, in the groups treated with EGF that is either free or released from the microparticle (MP:EGF), the wound area presents more reduction than in the control groups. After 7 and 11 days, the MP:EGF group shows a statistically significant decrease of the wound area (61.06$\pm$8.69%) in comparison with free EGF (51.46$\pm$10.29%). On days 7 and 11 after wound creation, the wound closure speed of the MP:EGF group was significantly higher than that of the other groups. It should be pointed out that free EGF is administered twice a week (75 $\mu$g dose of EGF), whereas the EGF released from the microparticles derives from a unique application. This entails an important advantage since the number of repeated injections to be made in the wound area decreases, and the probability of suffering adverse effects with respect to treatment also decreases.

Re-epithelialization

The animals were sacrificed with $CO_2$ on days 7, 11 and 17 for the histological analysis (Figure 5).

The degree of re-epithelialization was measured following the criterion established in Sinha et al. (Effects of steel scalpel, ultrasonic scalpel, CO2 laser, and monopolar and bipolar electrosurgery on wound healing in guinea pig oral mucosa. Laryngoscope 2003; 113(2):228-236). The areas where the lesion was located were extracted with the aid of a scalpel, fixed in 10% formaldehyde for 24 hours and stained with hematoxylin-eosin stain. Re-epithelialization after the seventh day was studied.

As shown in Figure 5, in the group treated with microparticles with EGF and in the untreated control group re-epithelialization is completed without irregularities on day 11. Complete re-epithelialization was not achieved in the remaining experimental groups until the end of the study. Furthermore, re-epithelialization had normal thickness only in the MP:EGF group, which does not occur when control or free EGF solutions are used. This entails an important advantage since healing is similar to physiological healing with the EGF released from the microparticles of the invention, the scar tissue being mature and physiologically well organized.

EXAMPLE 7. Microparticles Embedded in a Fibrin Matrix

Thrombin solution preparation: Aliquot thrombin at a concentration of 50 U/mL in a 0.1 % (w/v) BSA solution and 0.025 mM of $CaCl_2$. Store at -20°C.

Fibrinogen solution preparation: Weigh 30-60 mg of fibrinogen and add 1 mL of physiological serum (30 mg/mL). Leave at 37°C until it is dissolved.

Fibrin gel gelling: Add 0.5 mL of the fibrinogen solution to a test tube where the rhEGF microparticles are located. Add 0.125 mL of thrombin solution to it, rapidly collect it all and add to a 24-well plate. Gel in an oven at 37°C and wait until coagulation. The fibrin gel thus prepared contains 15-30 mg of fibrin.

Table 4 shows the percentage of wound closure as a function of time in days. Data shown as mean ± standard deviation.

Table 4. Percentage of wound closure and standard deviation.

|  | Day 1 | | Day 4 | | Day 8 | |
|---|---|---|---|---|---|---|
|  | % closure | SD | % closure | SD | % closure | SD |
| Control | 0.00 | 0.00 | 5.04 | 5.71 | 9.86 | 7.94 |
| Vehicle control | 0.00 | 0.00 | 1.75 | 6.98 | 14.49 | 8.81 |
| Blank MP control | 0.00 | 0.00 | 7.35 | 4.36 | 9.63 | 5.85 |
| Free EGF | 0.00 | 0.00 | 8.42 | 3.96 | 19.94 | 5.21 |
| MP EGF | 0.00 | 0.00 | 22.10 | 7.73 | 43.12 | 3.97 |
| Fibrin | 0.00 | 0.00 | 5.92 | 3.46 | 19.09 | 9.63 |
| MP EGF + fibrin | 0.00 | 0.00 | 17.91 | 1.74 | 29.36 | 4.34 |

As can be seen in Table 4, the EGF released from the microparticles embedded in a fibrin matrix maintains its biological activity *in vivo* and is able to promote wound healing (Table 4).

**Claims**

1. Microparticle comprising a polylactic-co-glycolic acid (PLGA) polymer, an alginate polymer and epidermal growth factor (EGF).

2. Microparticle according to claim 1, where the epidermal growth factor is recombinant human epidermal growth factor (rhEGF).

3. Microparticle according to claim 1 or 2 comprising between 82.7% and 99.897% (w/w) PLGA, between 0.003% and 1.5% (w/w) epidermal growth factor and between 1% and 5% (w/w) alginate relative to the total weight of the microparticle.

4. Microparticle according to any one of the preceding claims further comprising 0.1-10% (w/w) human serum albumin and 0.1-0.8% (v/w) polyethylene glycol relative to the total weight of the microparticle.

5. Microparticle according to any one of the preceding claims, **characterized in that** the epidermal growth factor is released for at least 30 days.

6. Microparticle according to any one of the preceding claims embedded in a fibrin matrix.

7. Pharmaceutical composition comprising a microparticle according to any one of claims 1 to 6 and a pharmaceutically acceptable carrier.

8. Microparticle according to any one of claims 1 to 6, or pharmaceutical composition according to claim 7, for its use as a drug.

9. Microparticle according to any one of claims 1 to 6, or pharmaceutical composition according to claim 7, for its use in promoting wound healing in an individual.

10. Microparticle or pharmaceutical composition according to the preceding claim, wherein the wounds are selected from the group consisting of diabetic foot ulcers, pressure ulcers, vascular ulcers and combinations thereof.

11. Kit comprising a microparticle according to any one of claims 1 to 6, and/or a pharmaceutical composition according to claim 7 to promote wound healing.

12. Method for preparing a microparticle according to any one of claims 1 to 5 comprising the following steps:

   a. Adding a solution of PLGA in an organic solvent (organic PLGA solution) to a solution comprising alginate and EGF (EGF and alginate solution), and mixing the organic PLGA solution and the EGF and alginate solution,
   b. Adding the emulsion obtained in step a) to an aqueous solution comprising surfactants and sodium chloride (aqueous solution), and mixing the emulsion obtained in step a) and the aqueous solution,
   c. Adding the emulsion obtained in b) to a solution comprising sodium chloride and calcium chloride (chloride solution), and mixing the emulsion obtained in step b) and the chloride solution,
   d. Extracting the solvent, and
   e. Isolating the microparticle.

13. Method according to the preceding claim, wherein the organic solvent of the organic PLGA solution is a mixture of dichloromethane and acetone (3:1).

14. Method according to any one of claims 12 or 13, wherein the aqueous solution of step b) comprises 5-10% (w/v) NaCl relative to the total volume of the aqueous solution and the chloride solution of step c) comprises 5-10% (w/v) NaCl in volume relative to the total volume of the chloride solution and 0.6 to 1.2 mM of $CaCl_2$.

15. Method for preparing a microparticle according to claim 6 comprising the following steps after the steps of the method according to any one of claims 12-14:

   f. Lyophilizing the microparticle isolated in step e), and
   g. Embedding the lyophilized microparticle obtained in step f) in a fibrin matrix.

**FIG. 1**

**FIG. 2**

FIG. 3

FIG. 4

**FIG. 5**

Europäisches
Patentamt
European
Patent Office
Office européen
des brevets

**EUROPEAN SEARCH REPORT**

Application Number

EP 12 38 2476

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 1 720 989 A (SHENZHEN TSINGHUA YUANXING BIO [CN]) 18 January 2006 (2006-01-18) * examples 1,2,5 * | 1-15 | INV. A61K9/00 A61K38/18 A61K9/06 A61K47/42 A61K9/16 C07K14/485 |
| X | EP 2 075 004 A1 (CT INGENIERIA GENETICA BIOTECH [CU]) 1 July 2009 (2009-07-01) * figure 2; example 4 * | 1-15 | |
| X,D | EP 1 987 817 A2 (CT INGENIERIA GENETICA BIOTECH [CU]) 5 November 2008 (2008-11-05) * figure 4; example 1 * | 1-15 | |
| A | DOWNS E C ET AL: "CALCIUM ALGINATE BEADS AS A SLOW-RELEASE SYSTEM FOR DELIVERING ANGIOGENIC MOLECULES IN VIVO AND IN VITRO", JOURNAL OF CELLULAR PHYSIOLOGY, WILEY SUBSCRIPTION SERVICES, INC, vol. 152, no. 2, 1 August 1992 (1992-08-01), pages 422-429, XP002072808, ISSN: 0021-9541, DOI: 10.1002/JCP.1041520225 * the whole document * | 1-15 | TECHNICAL FIELDS SEARCHED (IPC) A61K C07K |
| A | FERNANDEZ-MONTEQUIN J I ET AL: "Intralesional administration of epidermal growth factor-based formulation (Heberprot-P) in chronic diabetic foot ulcer: Treatment up to complete wound closure", INTERNATIONAL WOUND JOURNAL, BLACKWELL PUBLISHING LTD, UK, vol. 6, no. 1, 1 February 2009 (2009-02-01), pages 67-72, XP009143739, ISSN: 1742-4801, DOI: 10.1111/J.1742-481X.2008.00561.X * the whole document * | 1-15 | |

The present search report has been drawn up for all claims

| Place of search | Date of completion of the search | Examiner |
|---|---|---|
| The Hague | 12 April 2013 | Palma, Vera |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                    

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 12 38 2476

This annex lists the patent family members relating to the patent documents cited in the above-mentioned European search report.
The members are as contained in the European Patent Office EDP file on
The European Patent Office is in no way liable for these particulars which are merely given for the purpose of information.

12-04-2013

| Patent document cited in search report | | | Publication date | Patent family member(s) | | | Publication date |
|---|---|---|---|---|---|---|---|
| CN 1720989 | A | | 18-01-2006 | NONE | | | |
| EP 2075004 | A1 | | 01-07-2009 | AR | 063088 | A1 | 23-12-2008 |
| | | | | AT | 544462 | T | 15-02-2012 |
| | | | | AU | 2007304640 | A1 | 10-04-2008 |
| | | | | CA | 2665117 | A1 | 10-04-2008 |
| | | | | CL | 28622007 | A1 | 23-05-2008 |
| | | | | CN | 101573130 | A | 04-11-2009 |
| | | | | DK | 2075004 | T3 | 14-05-2012 |
| | | | | EP | 2075004 | A1 | 01-07-2009 |
| | | | | ES | 2386058 | T3 | 08-08-2012 |
| | | | | JP | 2010505773 | A | 25-02-2010 |
| | | | | PT | 2075004 | E | 02-05-2012 |
| | | | | RU | 2009116646 | A | 10-11-2010 |
| | | | | US | 2010136062 | A1 | 03-06-2010 |
| | | | | WO | 2008040260 | A1 | 10-04-2008 |
| | | | | ZA | 200902311 | A | 28-04-2010 |
| EP 1987817 | A2 | | 05-11-2008 | AR | 059234 | A1 | 19-03-2008 |
| | | | | AT | 437631 | T | 15-08-2009 |
| | | | | AU | 2007211753 | A1 | 09-08-2007 |
| | | | | BR | PI0707395 | A2 | 03-05-2011 |
| | | | | CA | 2640743 | A1 | 09-08-2007 |
| | | | | CN | 101400338 | A | 01-04-2009 |
| | | | | DK | 1987817 | T3 | 30-11-2009 |
| | | | | EP | 1987817 | A2 | 05-11-2008 |
| | | | | ES | 2330688 | T3 | 14-12-2009 |
| | | | | JP | 2009525288 | A | 09-07-2009 |
| | | | | KR | 20080096804 | A | 03-11-2008 |
| | | | | MY | 143742 | A | 15-07-2011 |
| | | | | PT | 1987817 | E | 26-10-2009 |
| | | | | SG | 169366 | A1 | 30-03-2011 |
| | | | | SI | 1987817 | T1 | 31-12-2009 |
| | | | | US | 2009220608 | A1 | 03-09-2009 |
| | | | | WO | 2007087759 | A2 | 09-08-2007 |
| | | | | ZA | 200806648 | A | 27-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007087759 A
- WO 9118999 A1

### Non-patent literature cited in the description

- **GOLDMAN R.** Growth factors and chronic wound healing: past, present and future. *Adv Skin Wound Care,* 2004, vol. 17, 24-35
- **CHU et al.** Nanotechnology promotes the full-thickness diabetic wound healing effect of recombinant human epidermal growth factor in diabetic rats. *Wound Repair Regen,* 2010, vol. 15, 499-505
- **MARIOKA-FUJIMOTO et al.** Modified enterotoxin signal sequences increase secretion level of the recombinant human epidermal growth factor in Eschsrichia coli. *J Biol Chem.,* 25 January 1991, vol. 266 (3), 1728-32
- **IGARTUA et al.** γ-Irradiation effects on biopharmaceutical properties of PLGA microspheres loaded with SPf66 synthetic vaccine. *European Journal of Pharmaceutics and Biopharmaceutics,* 2008, vol. 69 (2), 519-526
- **PURAS et al.** Encapsulation of Aβ1-15 in PLGA microparticles enhances serum antibody response in mice immunized by subcutaneous and intranasal routes. *European Journal of Pharmaceutical Sciences,* 2011, vol. 44 (3), 200-206
- **WASHINGTON C.** Particle size analysis in pharmaceutics and other industries: theory and practice. Taylor & Fancis, 1992
- **SALVADOR et al.** Combination of immune stimulating adjuvants with poly(lactide-co-glycolide) microspheres enhances the immune response of vaccines. *Vaccine,* 2012, vol. 30 (3), 589-596
- Human EGF Elisa development kit protocol. *Peprotech,* 2011
- Malvern Instruments user manual. 2009
- **ZHOU et al.** Effects of Leukemia Inhibitory Factor on Proliferation and Odontoblastic Differentiation of Human Dental Pulp Cells. *J Endod,* 2011, vol. 37 (6), 819-824
- **WALTERA et al.** Mesenchymal stem cell-conditioned medium accelerates skin wound healing: An in vitro study of fibroblast and keratinocyte scratch assays. *Experimental Cell Research,* 2010, vol. 316, 1271-1281
- **LI et al.** Research of PDGF-BB Gel on the Wound Healing of Diabetic Rats and Its Pharmacodynamics. *J Surg Res,* 2008, vol. 145 (1), 41-8
- **GALIANO ; MICHAELS.** *Quantitative and reproducible murine model of excisional wound healing. Wound repair and regeneration,* 2004, vol. 12 (4), 485-492
- **SINHA et al.** Effects of steel scalpel, ultrasonic scalpel, CO2 laser, and monopolar and bipolar electrosurgery on wound healing in guinea pig oral mucosa. *Laryngoscope,* 2003, vol. 113 (2), 228-236